# EUROPEAN PATENT SPECIFICATION

(11) **EP 1 315 509 B1**
(45) Date of publication and mention of the grant of the patent: **08.08.2007**
(21) Application number: 01965420.1
(22) Date of filing: 10.09.2001
(51) Int. Cl.: A61K 33/02, A61K 47/12, A61P 31/04

(54) **NITROGEN OXIDE FOR USE IN THE TREATMENT OF DRUG RESISTANT ORGANISMS**
STICKSTOFFOXID ZUR BEHANDLUNG THERAPIERESISTENTER ORGANISMEN
OXYDE D'AZOTE POUR L'UTILISATION DANS LE TRAITEMENT D'ORGANISMES RESISTANTS AUX MEDICAMENTS

(30) Priority: 08.09.2000 GB 0022084
(43) Date of publication of application: 04.06.2003
(73) Proprietor: ABERDEEN UNIVERSITY, Aberdeen AB2 1DY (GB)
(72) Inventor: BENJAMIN, Nigel, London N19 4PW (GB); ORMEROD, Anthony, Aberdeen AB15 8SG (GB)
(74) Representative: Lord, Hilton David
(86) International application number: PCT/GB2001/004048
(87) International publication number: WO 2002/020026

(56) References cited:
- WO-A-95/22335
- WELLER R ET AL: "A randomized trial of acidified nitrite cream in the treatment of tinea pedis" JOURNAL OF THE AMERICAN ACADEMY OF DERMATOLOGY, C.V. MOSBY, ST. LOUIS, MO, US, vol. 38, no. 4, April 1998 (1998-04), pages 559-563, XP002111730 ISSN: 0190-9622
- BOGDAN C C ET AL: "Reactive oxygen and reactive nitrogen intermediates in innate and specific immunity" CURRENT OPINION IN IMMUNOLOGY, CURRENT BIOLOGY LTD, XX, vol. 12, no. 1, 1 February 2000 (2000-02-01), pages 64-76, XP004257620 ISSN: 0952-7915
- FANG F C: "MECHANISMS OF NITRIC OXIDE RELATED ANTIMICROBIAL ACTIVITY" JOURNAL OF CLINICAL INVESTIGATION, vol. 99, no. 12, 12 July 1997 (1997-07-12), pages 2818-2825, XP002189642
- EHLERT K: "METHICILLIN-RESISTANCE IN STAPHYLOCOCCUS AUREUS - MOLECULAR BASIS, NOVEL TARGETS AND ANTIBIOTIC THERAPY" CURRENT PHARMACEUTICAL DESIGN, vol. 5, no. 2, February 1999 (1999-02), pages 45-55, XP001055322

## Description

The present invention relates to use of a nitrogen oxide generating composition in the manufacture a medicament for the treatment of antibiotic resistant organisms, and to formulations suitable for such use.

The emergence of bacteria which are resistant to antibiotic therapy is an increasing problem in clinical situations. Patients acquiring an infection by a resistant bacterium, such as methicillin resistant *Staphylococcus aureus* (MRSA), often have a longer duration of stay in hospital and/or are subject to isolation procedures, both of which contribute to increased health care costs and are inconvenient and distressing for the patient. Such infections can be life threatening and, indeed, the increasing prevalence of multiply antibiotic-resistant bacteria has led to higher mortality rates. In this connection, the term "antibiotic resistant bacteria" includes the organism known as methicillin resistant *Staphylococcus aureus* (MRSA) and other organisms and/or strains which are singly or multiply resistant to antibiotic therapy. References to MRSA which, itself, may be multiply antibiotic resistant, hereinafter, include references to other singly and multiply antibiotic resistant organisms or bacteria, unless otherwise specified.

Existing protocols for the elimination of localised infections of MRSA in, for example, the nose or in wounds, rely on the use of antibiotics such as mupirocin or neomycin. Chlorhexidine can be applied as a daily skin or scalp wash. Systemic antibiotics, such as fusidic acid or rifampicin, can be used, but their use is dependent on the sensitivity of the infecting strain and, in some cases, on the level of side effects observed. Recently, mupirocin resistant organisms have emerged at levels as high as 22-59% of organisms isolated from hospitalised patients.

Accordingly, there is an urgent need for alternative topical agents that do not rely on conventional antibiotics for treatment control of such potentially hazardous organisms in the community. Also, while mupirocin may be used on infected lesions, it is not suitable for large areas, such as burns or pressure sores.

The mechanism of MRSA resistance to antibiotics is complex and involves several components. For example, the cell wall of MRSA is unique, in that it contains penicillin binding proteins (PBP), such as PBP1, PBP2, PBP2' and PBP2a which bind penicillin into the wall of the bacteria, thereby preventing it from reaching its target. Accordingly, the penicillin neither affects the growth of nor kills MRSA. In addition, the cell walls of the MRSA organism are unusually thick, thereby preventing access to toxins.

Accordingly, there is a need for an alternative treatment for antibiotic resistant organisms.

Surprisingly we have now found that MRSA is sensitive to the reaction products of acidified nitrite, even at very low levels.

Thus, in a first aspect, the present invention provides the use of a nitrogen oxide generating composition in the manufacture of a medicament for the treatment and/or prophylaxis of a Methicillin Resistant S. *aureus* (MRSA) infection of a subject, wherein the medicament is for topical application and is provided as at least two components for admixture, either immediately prior to application or *in situ,*
essential elements, or reactants, for said nitrogen oxide generation being separately disposed in said components, such that there is substantially no nitrogen oxide generation until admixture.

The term "drug", as used herein, relates to a substance which has a static or cidal effect on an infection. In the case of bacteria, for example, such a substance is generally an antibiotic. The terms "drug resistant" and "multiply drug resistant" have the concomitant meanings.

It will be understood that reference herein to compositions are for use in the present invention.

The nitrogen oxide generating composition will typically comprise two or three essential reactants, these generally being a nitrite, an acid and/or a reducing agent. The nitrite, or suitable source therefor, will be provided in one component, while the acid and/or reducing agent may be provided in the other. Where there are both acid and reducing agent, these may be provided separately or together in one or two components of the medicament, but should not be provided with the nitrite, in order to avoid depletion of the nitrite prior to use. It will be appreciated that an "essential" reactant is one without which nitrogen oxide would not be generated and that such essential reactants should not be provided together within any one component where a nitrogen oxide generating reaction would result. In this respect, an essential reactant includes water, so that it is possible to mix nitrite and acid, in the absence of water, as a component. This is not generally preferred, however, as water may deliquesce, or otherwise contaminate the component, thereby leading to reaction.

It will be appreciated that the term "elements" is used in respect of an element of the composition for generating nitrogen oxide, rather than an element of the Periodic Table.

It will be understood that sufficient concentrations of the reactants need be provided in order to achieve generation of nitrogen oxide, but that levels only need be low, as low levels of NO generation are sufficient for the purposes of the present invention. Where an acid is used, this should be provided at such levels as to provide a pH of about 4.5 or below in the final medicament, at least immediately after admixture. However, pH requirements are discussed in more detail below.

Admixture preferably takes place *in situ,* in order to make as much use as possible of the oxides of nitrogen generated by the composition. Nevertheless, mixing prior to use is also envisaged, such as in the preparation of creams or ointments or handwashes, for example. In addition, the medicament may comprise one or more further components that do not necessarily comprise a reactant, but may comprise another constituent of the final medicament, such as a gelling agent, for example.

In some cases, it is desirable to intimately mix the components, so as to facilitate rapid nitrogen oxide production while, in other cases, it is sufficient merely to bring the components into contact one with the other, relying on diffusion to mingle the reactants, thereby leading to a greater length of time over which the composition is active.

It is envisaged that some generation of nitrogen oxide may occur prior to use, but conditions should preferably be chosen to minimise such occurrence, so as to maximise the amount of nitrogen oxide that can be generated *in situ.*

In another aspect, the present invention provides the use in the manufacture of a topical medicament for the treatment and/or prophylaxis of multiply antibiotic resistant bacteria on the human or animal skin which comprises a pharmaceutically acceptable source of NO/NO₂, said source being present in an amount sufficient to kill and/or prevent growth of the multiply antibiotic resistant bacteria on topical administration of the medicament to the skin, wherein the bacteria is Methicillin Resistant *S. aureus* (MRSA).

Nitric oxide (NO) is well known to have antimicrobial and wound healing effects [*c.f.* WO 95/22335; Hardwick et al., (2001), Clin. Sci., 100, 395-400], and has been shown to be useful in the treatment of a large number of exogenous conditions.

WO 95/22335 and Weller et al (J. American Academy of Dermatology, vol. 38, no. 4, April 1998) disclose the treatment of bacterial, viral or fungal infections by topical application of a Nitrite component and an acidifying component to produce the active NO. However, no mention of drug-resistant infections is made, Weller *et al* relates to tinea pedis specifically.
Bogdan, et al. (Curr. Op. Immun. XX, vol. 12, no. 1, 1 Feb 2000), discusses the general effect of active nitrogen intermediates in immunity. Fang, F. C. (J. Clin. Investigation, vol 99, no12, 12 July 1997), relates to the mechanisms of action of nitric oxide against microbes. Although various suggested mechanisms are proposed, as well as defence mechanisms by the microbes, there is no suggestion that the mechanism of action is such as to overcome multiple drug resistance. Ehlert, K (Curr. Pharma. Des.) highlights the problems with drug resistant *Staphylococcus aureus,* but again makes no suggestion that nitric oxide nmay be useful.

Nitric oxide is synthesised in the body in the endothelium and neurons, as well as in activated macrophages. On the skin, low levels of NO are produced by a combination of nitrite present in sweat and the skin's slightly acid pH. Although it is not known precisely what the method of action is as an antimicrobial agent/in wound-healing, it is speculated that NO serves to disrupt bacterial DNA, and to work at several levels also inhibiting metallo-enzymes and ribonucleases.

Nitric oxide is most conveniently produced by the reaction of nitrite with an acid. This results in the production of the molecular form of nitrous acid, which readily dissociates into a molecule of water and a molecule of dinitrogen trioxide, the latter, in turn, dissociating to form nitric oxide and nitrogen dioxide. The reactions are shown below.

NO₂⁻ +H⁺ ↔ HNO₂

2HNO₂ ↔ N₂O₃ + H₂O

N₂O₃ ↔ NO + NO₂

In the presence of a reducing agent, such as ascorbic acid, the reaction of dinitrogen trioxide to form nitric oxide is more efficient, and can be represented, for example, as follows:

N₂O₃ + C₆H₈O₆ → 2NO + H₂O + C₆H₆O₆

Nitrous acid and, subsequently, NO/NO₂ may suitably be generated, for example, by the action of an acid on a nitrite, particularly where the resulting salt is insoluble. An example is the reaction of barium dinitrite and sulphuric acid, which yields the insoluble barium sulphate and a solution of nitrous acid which may then dissociate as exemplified above.

It will be appreciated that, whilst any suitable source of nitrogen oxides providing at least a fraction of nitric oxide may be employed in the present invention, it is generally preferred that any nitrogen oxides be generated in accordance with one or more of the above reactions.

In the context of the above reactions, it will be appreciated that a pharmaceutically acceptable source of NO/NO₂ is one that yields either or both compounds, when topically applied, in therapeutically useful amounts. Suitable sources are described in more detail below, but will generally comprise a nitrite and an acid mixed *in situ,* or shortly before application, to avoid wasted generation of the gaseous products. Indeed, the reaction generally goes to completion within minutes, and solutions of NO and NO₂, as such, are not stable for any period, so that they must be used rapidly after preparation. Once applied, however, it has surprisingly been found that there is still measurable potency, even after 24 hours (*c.f.* Example 2, below).

WO 95/22335 discloses that acidified nitrite, such as described above, can be used to liberate gaseous nitric oxide (NO) and nitrogen dioxide (NO₂) onto the skin as a topical antimicrobial therapy for sensitive (*i.e*. non-resistant) bacteria.

NO/NO₂ as therapeutic agents are less specific than antibiotics, in general, so that the target organism may be affected at several sites and/or by several mechanisms. These may include disruption of DNA and RNA synthesis, thus inhibiting cell replication, inhibition of cell respiratory enzymes and disruption and inactivation of enzymes, proteins and lipid membranes, by, for example, oxidation or nitrosation.

In this respect, resistant organisms are highly sophisticated, having developed multiple mechanisms for withstanding, in many cases, all known specific and broad spectrum antimicrobial agents, such as antibiotics. In addition, these bacteria have developed many other additional ways of avoiding being killed, including cells being less permeable and having thickened walls. Indeed, those skilled in the art consider that a contaminated wound, infected with MRSA or other resistant organisms, is likely to remain that way until the wound heals - i.e. nothing works.

However, we have now shown, through both *in vivo* and *in vitro* work, that methicillin resistant *Staphylococcus aureus,* for example, can readily be killed using NO/NO₂, particularly when derived from an acidified nitrite, by the admixture on the skin of a source of nitrites and an acid, preferably an organic acid. As MRSA is difficult, or occasionally impossible, to treat with any other antibiotics, the finding that NO and NOz are effective in the treatment of MRSA infections is quite unexpected.

It is nothing short of astonishing, that MRSA is susceptible to something as simple as the compositions of the present invention, and that all strains tested, to date, are at least as susceptible as sensitive strains. Indeed, what is surprising is that some strains of MRSA are actually more sensitive than MSSA (methicillin sensitive *S. aureus),* making the invention particularly useful in the treatment and prevention of MRSA infections.

Surprisingly, we have found that efficacy *in vivo* can be enhanced by the use of the very antibiotics to which the organism is resistant. For example, in a clinical situation involving fusidic acid resistant MRSA, a complete cure was effected using a combination of topical acidified nitrite and systemic fusidic acid. Thus, a further benefit of the compositions of the present invention is that patients treated therewith may be treated with other antibiotics, for example penicillin and cephalosporins, to which they did not previously respond.

As noted above, while the mechanism of the antibiotic activity of NO has not been fully elucidated, there was no reason to expect it to work synergistically with other antibiotics. It is known that interfering with fem factors, for example, tends to restore MRSA to its antibiotic sensitive state (Ehlert, K., Methicillin Resistance in Staphylococcus aureus, Curr. Pharm. Des., 1999; 5: 45-55), but NO has only previously been associated with the disruption of bacterial DNA, and the inhibition of metallo-enzymes and ribonucleases.

Accordingly, in a preferred aspect, the present invention provides the use of a nitrogen oxide generating composition in the treatment and/or prophylaxis of an Methicillin Resistant S. *aureus* (MRSA) infection, characterised in that said treatment or prophylaxis further comprises use of an antibiotic suitable for use against non-resistant strains of the said infection. More particularly, the antibiotic is preferably one to which the infection was resistant until treatment with NO/NO_{2.} The antibiotic may be administered concurrently with the NO generating source, or subsequently, and may be administered topically or systemically, as desired.

An additional benefit of treatment using NO is that it is a gaseous compound which can diffuse readily into the skin or wound to treat the infection, and does so more readily than conventional antibiotics, which tend to rely on relatively large molecules in solution. Prevention of wound infection is particularly important, as this prevents potentially fatal systemic infection.

As used herein, the term "skin" includes the mucosae, for example, as well as such discontinuities as wounds and lesions. In addition, in the present invention the terms NO and/or NO₂ also include other nitrogen intermediates. It should be noted that although the present invention is mainly directed towards use on the skin, it is possible that antibiotic resistant organisms found on other bodily surfaces could be killed or their growth inhibited by the compositions of the present invention. Disinfection of non-bodily surfaces is described below.

The wide range of activity of compounds of the present invention against MRSA, mean that it can also be used as a disinfectant for hands and as a prophylactic agent in the treatment of surgical wounds and in other high risk environments, e.g. burns, immuno-compromised individuals and in the treatment of asymptomatic carriers, for example, nurses and medical staff.

*In vitro* tests have established that all coagulase-negative Staphylococcus aureus organisms are susceptible to NO. What is particularly surprising is that MRSA can actually be more sensitive to NO than normal, sensitive strains. What is more, NO is effective in surprisingly small amounts. Thus, it is not essential to produce large amounts of gaseous NO, provided that sufficient is present to kill or control MRSA. Thus, compositions of the present invention may be used on wounds or in nasal passages, for example, or simply as a standard hand wash.

More generally, the compositions of the present invention may be applied in any suitable manner to treat or prevent infection Methicillin Resistant *S. aureus* (MRSA,). Suitable formulations will depend on the location or area to be treated, and the desired characteristics.

Suitable dermal conditions for treatment with the present invention include, for example, orthopaedic pin wounds, pressure sores, catheter associated punctures, burns and surface traumas. Skin penetration, such as around pin sites, and catheter associated infections may be treated or prevented both before insertion and after removal.

In particular, compositions of the present invention may be used, for example:
1) On open wounds, as it has surprisingly been found that formulations, such as creams, of the invention are not irritant to such wounds;
2) For MRSA prophylaxis in at-risk patient care situations;
3) As synergistic agents with conventional antibiotics, as it has surprisingly been found that, following nitric oxide therapy, conventional antibiotics are able to remove any remaining infection; and
4) In situations where acid levels need not to be lower than a pH of about 2.

Under experimental conditions, incubation for two hours at pH 3.5 with 1 mmol nitrite completely inhibits MRSA growth and kills the organism. At pH 4.5, MRSA is inhibited similarly by two hours incubation with 10 mmol nitrite. Thus the range of pH and nitrite concentrations are significantly wider than previous prior art indications.

Thus, in preferred embodiments, the present invention relates to the use of nitric oxide (NO) and/or nitrogen dioxide (NO₂), preferably from acidified nitrite, more preferably still at a pH below 5 and, more preferably still, at a lower pH, for example pH 4.5 or 4, in the manufacture of a medicament for treating Methicillin Resistant S. aureus (MRSA).

The pharmaceutically acceptable source of NO and/or NO₂ is preferably a pharmaceutically acceptable nitrite and a pharmaceutically acceptable acidifying agent admixed therewith on, or adjacent to, the skin, the sources being held separately until, or just prior to, administration.

Preferably, the pH immediately after admixture is below 7, but more preferably below 5, and particularly preferably below 4. However, it is also preferred that the pH be above 2, in order to avoid irritation caused by excess acidity.

In general, it is preferred that the nitrite comprises alkali metal or alkaline earth metal nitrite in amounts as small as 0.005%, in solutions, for example, and as high as 12%, or even higher, in creams, for example. A preferred range is from 0.005 to 10% by volume of the source. The acidifying agent is preferably an organic acid. Preferred amounts of acidifying, agent are from as low as 0.5% to as high as 22%, or higher, with a preferred range being from 0.5 to 15% by volume.

In an alternative aspect, the present invention provides the use in the manufacture of a topical medicament for the treatment of methicillin resistant *Staphylococcus aureus* (MRSA) infections of a pharmaceutically acceptable acidifying agent and a pharmaceutically acceptable source of nitrites, both said agent and said source being disposed respectively and separately in a pharmaceutically acceptable carrier or diluent therefor, said acidifying agent being present in an amount sufficient to induce an acidic pH on administration of the source and the agent to the skin.

In a yet further aspect, the present invention provides the use in the manufacture of a composition for the induction of cell death or prevention of cell replication of Methicillin Resistant *S. aureus* (MRSA) of a pharmaceutically acceptable acidifying agent and a pharmaceutically acceptable source of nitrites, both said agent and said source being disposed respectively and separately in a pharmaceutically acceptable carrier or diluent therefor.

The compositions of the present invention may be any that are suitable to provide nitrogen oxides. Without being restricted by theory, it is believed that nitric oxide (NO) is the active. Accordingly, it is preferred that at least a portion of the nitrogen oxides generated by the composition should be nitric oxide. More preferably, the proportion of nitric oxide generated by the compositions of the present invention is preferred to be at least 50% and, more preferably, at least 80%. Where the only acid used is a reducing acid, then this proportion may rise to anything up to 100% nitric oxide content of the nitrogen oxides generated.

In the above discussion, it will be appreciated that the nitrogen oxides, of which nitric oxide forms a portion, do not include dinitrogen trioxide, as this is regarded as an intermediate in the formation of nitric oxide and nitrogen dioxide.

By "nitrogen oxide generating" is meant that compositions of the present invention serve to release nitrogen oxide *in situ,* i.e. at the site of infection, or possible infection. At its simplest, this may comprise an ointment or gel or, indeed, any other suitable, topical vehicle, in which gaseous nitric oxide has been dissolved, for example, and which, once applied, releases nitric oxide.

Given that only small amounts of nitric oxide are required in order to be effective, then it does not matter if nitric oxide escapes other than at the site of application. Even only very small amounts are sufficient to have a cidal or static effect.

The nitrogen oxide generating composition may take any suitable form. However, it will be appreciated that, where the generation of nitrogen oxides is active, then the reactants should be kept separate, one from the other, until nitrogen oxide is actually required. Although this is generally a preference, it need not necessarily always apply. For example, an occlusive patch may be constructed with a gel, or matrix, into which nitrogen oxide generating ingredients are loaded, the patch then being protected by a suitable webbing. A suitable gel is a hydrogel.

Preferably, the matrix or gel is adhesive, and the strength of the adhesion is sufficient to overcome any tendency of the nitrogen oxide to escape and push away the webbing, although it will be appreciated that the strength of the adhesive should not be such that the webbing cannot be satisfactorily removed to allow application of the patch. Further, it is preferred to provide suitable stabilisers, such as chelating agents, in the gel or matrix, in order to prolong the life of the nitric oxide or to reduce the rate at which it is produced. Additionally, as nitric oxide is more soluble in non-aqueous and lipid substances, the addition of such substances to the treatment may prolong the activity and delivery of nitric oxide to the site of application.

Nevertheless, compositions already comprising free nitric oxide will not generally be stable for any great length of time, and should preferably be used by the patient as soon as possible after preparation.

More preferred is to provide the compositions of the present invention in multiple parts. These parts may each, separately, comprise actives or reactants, which, when mixed, serve to generate nitrogen oxides. Thus, a first composition may comprise a suitable nitrite provided in a suitable vehicle. A second composition may comprise a suitable acid. The two compositions can then be mixed, preferably intimately, and then applied to the site, or may be mixed *in situ.* Although it is generally desired to minimise the number of components that it is necessary to mix in order to achieve the final nitrogen oxide generating composition, it will be appreciated that any number may be provided. In particular, it may be preferred to provide a third composition comprising a reducing acid, for example. However, where a reducing acid, such as ascorbate, is used, then it is generally preferred to either use it as the acid, in its own right, or to provide it together with the primary acid in a separate composition from the nitrite.

Although the present invention is generally illustrated herein in respect of two compositions being mixed to provide the final, nitrogen oxide generated composition, it will be appreciated that such references include references to more than two initial compositions, unless otherwise apparent, or indicated.

Compositions of the present invention may comprise any suitable vehicles for mixing. What is important is that the acid and the nitrite, or nitrite precursor, be able to react in such a manner as to generate the desired nitrogen oxides. Thus, at least one of the initial compositions providing the final composition should preferably comprise an aqueous component, in order to allow the nitrogen oxide generating reaction to take place. More preferably, both of the initial compositions should comprise aqueous components to facilitate the mixing of the ingredients although, where it is desired that the ingredients should only react slowly, the amount of water may be minimised in one or both of the initial compositions.

There is no restriction on the types of initial compositions that may be mixed in order to achieve the final composition, provided that the final composition serves to generate nitrogen oxide. In this respect, and throughout, it will be appreciated that reference to "nitrogen oxides" includes reference to 100% nitric oxide.

For example, the initial compositions may be in any suitable form, such as liquid, gel or solid although, where one is solid, then the other is preferably liquid or gel. Solids are not generally preferred. In the category of liquid are included solutions, suspensions and colloids, and such considerations also apply to gels, which, as used herein, generally comprise any state between liquids and solids.

More particularly, reference to gels herein also includes reference to other semi-solid states, such as creams, ointments, tinctures, soft waxes and lotions, although the latter may fall under liquids, depending on the properties thereof. It will be appreciated that there is no specific exclusion, provided that the liquid, gel, or solid serves as a vehicle for the active.

Solid vehicles may include matrices in patches, for example.

The initial compositions may suitably be mixed, either before application or *in situ,* in order to provide the final composition to generate nitrogen oxides. Such mixtures may be straightforward gel/gel mixtures, for example, which can then be applied to the skin, and left in place. They may also comprise two liquids, two gels, or a liquid and a gel, which, between them, form a gel, for example, or otherwise form a protective environment to generate, hold and dispense nitric oxide.

In one preferred embodiment, a gel may be applied to the site and then a patch, such as a plaster, carrying a matrix containing the other active is applied over the gel and, once in contact, the actives slowly interact to generate nitrogen oxide.

In another preferred embodiment, the matrix of the patch, or plaster, is non-aqueous but hydrophilic, and contains a mixture of the actives in substantially dry form. In this case, the term "dry form" may include crystals incorporating water of crystallisation, for example. Thus, although both of the actives are present in the matrix of the patch, or plaster, they cannot react in the absence of suitable quantities of water. Where it is desired to apply the patch, or plaster, any protective webbing can be removed and a suitable quantity of water, such as a few drops, can be applied to the matrix to activate the active ingredients. The activated patch may then be applied to the skin to allow the nitrogen oxides generated to have their effect.

Activation of a patch may be effected by addition of a solution of one active component to a patch already comprising a matrix, such as a hydrogel, containing the other active. It will be appreciated that the matrix should not already be so saturated with water that the further solution of active is not readily taken up. Indeed, the matrix may be substantially dry, as described above.

This principle of providing substantially dry compositions to which water is added may also apply to other compositions. In such cases, it will be appreciated that the term "dry" applies to the water content, so that, whilst a composition may be a gel, for example, the water content will be extremely low, such as 1%, or even lower. It is preferred that such compositions are substantially anhydrous.

In one embodiment, the nitrite and acid are provided as powders to be applied direct to the site. While it is possible for water to be sprayed onto the resulting area, it is preferred to use this sort of mix on areas which are already moist, such as through sweating, or on wounds or lesions that are naturally moist or exuding, such as burns, open sores and open wounds. The moist environment then provides the necessary water for the reaction to start.

Although, at its simplest, this form of treatment simply comprises two separate powders, one nitrite and one acid, it will be appreciated that it may be desired to incorporate other materials, such as desiccants, bulking agents, gellants and preservatives.

The active ingredients of the compositions of the present invention may be present in any suitable quantities, as will be apparent to those skilled in the art. In general, it is preferred that the quantity of nitrite is approximately of 0.5 to 30%, by weight, of the final composition. More preferably, the amount of nitrite, or its precursor, is 1 to 15% and, particularly, 1 to 10% or 5 to 10%. In creams, it is envisaged that an upper limit is about 10%, although suitable formulation may permit higher levels.

It is preferred that the acid be present in at least stoichiometric amounts by comparison to the nitrite, or its precursor. More preferably, the acid is present in a stoichiometric excess, sufficient to ensure an acidic environment for a sufficient quantity of the nitrite to generate nitric oxide. Although it is not necessary for the whole of the nitrite to generate nitrogen oxides, it is generally inefficient to allow too much of the nitrite to go unreacted, and it is preferred that the majority of the nitrite be converted to nitrogen oxides.

In general, it is preferred that the acid be present in sufficient quantity that the final composition be at a pH of 5, or below, especially pH 4, or below. However, the nitrogen oxide generating reaction may take place a higher pH's, especially in the presence of excess reducing acids, so that it will be appreciated that the pH of the final composition does not form an essential part of the present invention.

The nature of the nitrite, for simplicity's sake, will generally be inorganic and at least partially soluble in water. Preferred are the alkali metal nitrites and the alkaline earth metal nitrites, although other suitable nitrites, such as the transition metal compounds, may also be used, subject to suitability, especially solubility. In particular, the sodium, potassium and barium compounds may be used, the sodium compounds generally being preferred from the point of view of expense and availability.

Suitable acidifying agents include inorganic acids but, owing to their general pharmaceutical unacceptability, are not generally preferred. Thus, more preferred are the organic acids, especially those capable of forming a solution with water and yielding a pH of 4 or below. Such acids include formic acid, acetic acid, malic acid, lactic acid, citric acid, benzoic acid, tartaric acid and salicylic acid, and it will be appreciated that this list is inclusive, rather than exclusive. Other suitable agents are reducing agents. These are not necessarily acids, and suitable reducing agents include ascorbic acid and ascorbyl palmitate which do not necessarily form such acidic solutions, but which are reducing acids and have the advantage of increasing the amount of nitric oxide generated, and which may also serve to stabilise the nitric oxide, once generated. The two types of agent may be used together, if desired.

Owing to the advantageous qualities of the reducing acids, in one embodiment it is preferred to provide a reducing acid in addition to the primary acid when forming the final composition. There is no particular proportion of reducing acid, although it is preferred that this be between 5 and 40% of the primary acid and, more particularly, between 10 and 20%.

Compositions of the present invention may be made by any suitable means. Where the compositions comprise aqueous components, then it is generally preferred to dissolve the active ingredients in water, or an aqueous preparation, which may then be kept separate from the other actives until required. Dry formulations may be made up substantially complete, save for the addition of water, which is added when it is desired to activate the composition.

Where the final composition comprises liquids or gels, these may be applied by any suitable means, including manual mixing. Other means may comprise a double barrelled syringe or a dual actuated dispenser, for example, with final mixing by a finger or spatula, or any other means appropriate.

Conveniently, the pharmaceutically acceptable nitrite comprises 0.005 to 10% by volume of alkali metal (e.g. sodium) nitrite, but any other effective alkali metal or alkaline earth metal nitrite source will do. A suitable range for the alkali metal or alkali earth metal is 0.005 to 5% by volume. Another is 3 to 8% by volume. A suitable range for the pharmaceutically acceptable acidifying agent, preferably an organic acid, is 0.5 to 15%, preferably 3 to 10%. Another suitable range is 1 to 9%. Conveniently the organic acid is citric acid or other nonreducing organic or inorganic acid.

Citric acid and sodium or potassium nitrite are used in a ratio of 1.2 : 1 to 2.5 : 1, preferably about 1.5 : 1 or 2:1 or inbetween, by weight or by molar ratio, in preferred formulations of the present invention. Particularly preferred ranges are 13.5% citric acid and 9% sodium nitrite or 3% sodium nitrite and 4.5% citric acid which may, along with the above, be disposed in creams, ointments, lotions, washes, emulsions, aqueous solutions etc. which serve as pharmaceutically acceptable carriers or diluents, so long as the acids and the nitrites are admixed at the environment of use.

When a reducing acid such as ascorbic acid is used, the effective pH may rise from above pH4 to as high as pH7, although the speed of reaction of the nitrite source and the acid (in this case organic acids) may be slow. Other sources of NO and NO₂ known to those in the art may be employed.

The composition of the invention may be applied to skin which is infected with resistant bacteria, or may be used as a prophylactic agent where infection is expected, for example, in a skin infection which will not respond to antibiotics, or in high risk patients likely to succumb to antibiotic resistant infections, or for clinical personnel who may be exposed to infected skin. Although it is generally preferred to treat such organisms as bacteria, it will be understood that normally resistant fungal, e.g. certain candida strains, infections will also be suitable for treatment or prophylaxis in accordance with the present invention. The application to sensitive body parts, such as nasal passages and other mucosae and wounds, is possible, owing to the feasibility of using low concentrations of active material.

The nitrite donors, particularly in the form of organic or inorganic salts, can give rise to free gases and/or an ionised form of NO or NO₂ moiety. These may be disposed in a pharmaceutically acceptable carrier or diluent such as a cream, ointment or aqueous solution. Similarly an acid which is adapted for administration with the source may be organic or inorganic and may be disposed substantially in a pharmaceutically acceptable carrier or diluent such as creams, ointments, lotions, washes, emulsions, aqueous solutions.

It is an advantage of the present invention that very few, if any, excipients are required. Indeed, preferred formulations of the present invention comprise sodium nitrite and citric acid, and it is generally desirable to keep excipients to a minimum, in order not to interfere with the NO/NO₂ generating reaction, or to acidify the nitrite, which would substantially reduce the shelf life of the nitrite component. Thus, where the nitrite component is stored in an aqueous condition, it is preferred to keep it at a pH of above 5.5 and, normally, at least 7, preferably about 7.5 or 8, until use. The acid component may be suitably prepared in order to take such pH of the nitrite into account on mixing.

It is generally preferred to dissolve sodium nitrite in water, optionally adding a preservative, such as propylene glycol, as a co-solvent, or preferably polyethylene glycol, which is generally less irritant. Both are miscible with water. Glycerin may be added as a thixotropic agent, for example. The product may also be in the form of a mild soap. A preferred formulation is a standard pump action spray, with or without propellant, for application to wounds.

For example, the compositions of the present invention may be applied as hand or body washes, in which case a nitrite, suitably sodium or potassium nitrite, may be dissolved in water as a first preparation. A suitable acid, such as citric or salicylic, may be provided in a second aqueous solution, and the two solutions disposed in a twin barrelled dispenser, for example. Turning a tap, or otherwise dispensing the solutions, then provides amounts of both to the user, optionally in one stream, to serve as a hand-wash. Rubbing the hands serves to further mix the solutions, thereby releasing NO.

It will be appreciated that either or both solution may be thickened, or provided as a liquid soap. Indeed, the nitrite may even be provided as a solid soap, with hands being washed under a dilute acid stream.

It is also preferred to provide the compositions of the invention for use in spray form. Similar considerations apply for mixture as for the solutions, twin reservoirs providing aerosols, for example, of the solutions. One or both may suitably comprise thixotropic agent(s) so that the resulting mix does not simply run off the treatment area.

Such sprays may also be useful in nasal applications. In such cases, it is generally preferred to use reducing acids, especially ascorbate or ascorbic palmitate, as the acidifying agent, so that higher pH's, such as about pH 5, can be used so as not to irritate the sensitive nasal mucosae.

It is particularly preferred to use the compositions of the invention on large areas, such as burns and pressure sores, for example. Thixotropic sprays may be used in such cases, or the compositions may be applied as gels, creams, unguents, ointments or other non-solid forms, the nitrite and acid not necessarily being provided each as a similar formulation type. For example, a nitrite gel may be applied to the area which is then sprayed with a suitable solution of citric or other acid. This may then be rubbed into the cream, or simply left to diffuse.

Foams, gels and sprays are preferred formulations of the present invention. These may be used in the treatment or prophylaxis of MRSA, and may be used in the prevention of colonisation, such as in post-surgical wounds.

Generally suitable formulations or dosage forms for use in the present invention are illustrated in the following table:

| **Formulation type** | **Details** |
|---|---|
| Cream | Water in oil base |
| Aqueous solution | Water base |
| Gel | Aqueous based |
| Ointment | Liquid paraffin base |
| Lotions | Aqueous base & mineral oil (liquid paraffin) |
| Handwashes | Aqueous base |
| Propellant Spray | Aqueous base & propellant gas (optionally containing alcohols) |
| Foam | Surfactant included |
| Emulsion | Oil in water base |

It will be appreciated that, in the case of aqueous solutions, for example, these can be presented as a spray or a wash, and that, if desired, excipients may be added to alter viscosity. Similar considerations apply to handwashes, in general.

It will be appreciated that the compositions of the present invention may be used to disinfect the skin or other objects such as, for example, surgical instruments or work surfaces. The pharmaceutically acceptable acidifying agent and a pharmaceutically acceptable source of nitrites are kept separately disposed until they are admixed at the environment of use.

The invention will now be described by way of illustration only, with reference to the following Example.

### EXAMPLE 1

This study was to assess whether NO/NO₂ produced by an acidified nitrite is able to eradicate MRSA from colonised wounds. The protocol was approved by the local Grampian Combined Ethical Committee and Infection Control Committees.

Hospitalised patients with an MRSA positive wound culture and negative throat and/or sputum culture were recruited into the study. Pregnant and lactating females and those with only nasal carriage of MRSA were excluded from the study, as were those taking systemic antibiotics. In addition, as noted above, when an acid, particularly an organic acid, and a nitrite, for example sodium nitrite, are mixed together they react to release nitric oxide (NO) and nitrogen dioxide (NO₂).

The study was limited to treating infections in specific target areas of the skin, e.g. wounds. In use, creams comprising the nitrite source (*i.e.* 3% sodium nitrite) and 4.5% citric acid were admixed on the wound or lesion twice daily for five days, swabs were then taken and repeated after 48 hours, and the results tabulated. After admixture a permanent absorbent dressing was placed over the admixture and the wound.

Swabs of the nose, throat, axillae, perineum and any other unhealed wounds were routinely swabbed to assess eligibility for the study and these swabs were repeated at baseline, day five of treatment, and 2 and 4 days after stopping therapy to assess recurrence. Those who developed nasal carriage of MRSA during the study were treated with mupirocin.

The first isolate of S. aureus and MRSA from each patient was typed and stored as a representative sample of the infection from that patient. On re-culture, resistance to methicillin was confirmed. Each isolate was tested for sensitivity to acidified nitrite using the following 96 well plate assay method.

The isolates were cultured in appropriate growth media until they reached a steady state for their rate of growth. Experiments were carried out in sterile 96 well plates. Potassium nitrite solutions were prepared at pH 7 and titrated to give final nitrite concentrations in the media wells of 0, 0.01, 0.03, 0.1, 0.3, 1, 3, 10 and 30 micromoles per ml, in rows of the plates. The pH was adjusted in a column-wise distribution to a pH of, respectively, 1.7, 2, 2.3, 3, 3.3, 3.6, 4, 4.5, 5, 5.5 and 6. Identical inocula were then inoculated into each well and subsequent bacterial growth counted by reading optical density. Each experiment was repeated 5 times. Seven MRSA isolates were compared to a standard non-resistant *Staphylococcus aureus.* Inhibition and killing of MRSA increasing proportionately to the increase in hydrogen or nitrogen ions, by linear regression analysis, implies that acidification of nitrite is responsible for the inhibition of, or killing of, MRSA.

The results of the above identified trials are shown in Table 1 below.

Colonisation of the wound, with negative swabs elsewhere, was confirmed in nine patients, seven of whom had more than one colonised wound. After treatment with acidified nitrite, three patients were cleared of infection without recurrence after five days. Three showed a partial response (clearance of one wound). Out of seventeen infected wounds eight were cleared of MRSA colonisation. There were five patients that developed positive swabs on other untreated body sites such as nose and throat during the investigation, although negative at the start. This occurred in two patients who responded (i.e. full clearance from all wounds with no recurrence), in two who did not respond, and in one showing a partial response.

**Table 1**

| | | | | | Assay at Days | | | | |
|---|---|---|---|---|---|---|---|---|---|
| | | | | | 0 | 5 | 7 | 9 | |
| Age | Sex | History | Wounds | Sites per patient | | | | | Other sites |
| 83 | F | Vasculitis on Prednisolone & azathioprine | Leg ulcers | 1 | + | + | + | + | Negative |
| | | | | 2 | + | + | - | - | |
| 74 | F | | Pressure sores | 1 | + | + | + | + | Positive |
| | | | | 2 | + | + | + | + | |
| 90 | F | | Amputation | 1 | + | - | - | - | Negative |
| 85 | F | | Orthopaedic pin sites | 1 | + | - | - | - | Negative |
| | | | | 2 | + | - | - | - | |
| 84 | M | Peripheral vascular disease | Gangrene of the toe | 1 | + | + | + | + | Negative |
| 29 | F | Crohn's disease Cyclosporin & Prednisolone | Parastomal pyoderma Gangrenosum | 1 | + | + | + | + | Positive |
| | | | | 2 | + | + | + | + | |
| 79 | F | Pemphigoid Prednisolone | Infected blisters | 1 | + | - | - | - | Negative |
| | | | | 2 | + | - | - | - | |
| | | | | 3 | + | - | - | - | |
| 68 | F | End stage renal failure | Calciphylaxis Ulcers on legs | 1 | + | - | - | + | Positive |
| | | | | 2 | + | + | + | + | |
| 74 | M | | Orthopaedic pin sites | 1 | + | - | - | - | Positive |
| | | | | 2 | + | + | + | + | |

It was not possible to achieve 100% clear up of resistant infections, owing to the limited nature of the clinical trial. For ethical reasons, it was only possible to treat selected areas of any one patient. Accordingly, it was possible that another infected locus could reinfect an area that had been successfully treated in accordance with the present invention. Nevertheless, it is clear that the present invention, even in such restricted conditions, is effective in the treatment of drug resistant infections.

Thus, it can be seen that the invention is useful in the treatment of MRSA by NO and/or NO₂, and to methods for improving the healing of wounds and lesions comprising MRSA, the lesions or wounds being on the skin. The invention is also useful, therefore, in the disinfection of skin which may have been in contact with a source of MRSA, by application thereto of NO and/or NO₂, particularly when produced by the administration of a source of nitrites with an acid at the environment of use.

### EXAMPLE 2

### Sensitivity of MRSA and MSSA to NO

The objectives of this study were to investigate the antibacterial activity of acidified nitrite against MRSA and MSSA (methicillin sensitive *S. aureus*). This was assessed through measurement of the Minimum Inhibition Concentration (MIC) and Minimum Bactericidal Concentration (MBC) of acidified nitrite in relation to a known MSSA strain and various MRSA strains, taking into account the relationship between MIC and pH, and the relationship between MBC, pH, and exposure time.

### Methodology

### Bacterial strains used

Seven clinically isolated methicillin-resistant *Staphylococcus aureus* (MSRA) strains, (A695, A713, A852, A977, A992, A1124, and A1350), one methicillin-sensitive *Staphylococcus aureus* (MSSA) and an MRSA control strain were obtained from the Department of Microbiology, Aberdeen Royal Infirmary (Aberdeen, Scotland UK). The MRSA isolates were derived from the initial cultures from MRSA patients treated in the pilot study of Example 1. Strains were kept in protected vials (Bacterial Preservers, Technical Service LTD, UK) at -24°C.

### Preparations of growth curve and calibration curves of turbidity versus cell density

### Preparations of growth curve

A single colony from nutrient agar (cultured over night at 37°C) was inoculated with 75ml nutrient broth in a 250ml flask. The flask was resealed with a foam stopper and incubated on a shaker at 120 rpm for 18 hours at 37°C. A 240µl sample of bacterial culture was taken at baseline (0hr) and thereafter every 2 hours and transferred into a well of micro well plate until steady growth phase was reached. Optical density was then measured using a micro well plate reader (Dynatech Laboratories MRX). Three repeat experiments were carried out for each strain studied.

### Preparation of calibration curve of numbers of colony forming units (cfu's) versus cell turbidity

Each bacterial strain was cultured as described above until stationary phase was reached. The optical density was measured at 570 nm using a micro well plate reader (Dynatech Laboratories MRX) by pipetting 240 µl of bacteria suspension into the well of micro well plate. A dilution series was made using nutrient broth (5x10⁻¹, 2x10⁻¹, 1x10⁻¹) and the corresponding optical densities were measured. Two further dilution series were made starting from 5x10⁻¹ and 1x10⁻¹ suspensions by transfer of 24 µl of suspension into 216 µl nutrient broth in a well of micro well plate, down to dilution factor of 10⁻⁸. Three drops of 10 µl of cell suspension at different dilution steps were pipetted onto nutrient agar plate. Plates were incubated at 37°C for 24 hours. The numbers of cfu's were counted. The numbers of bacteria in the original suspensions were calculated according the dilution factor.

A calibration curve was produced for each strain, relating the numbers of cfu's to the optical density at 570 nm. This curve was then used for all the further experiments to produce standardised cell inocula of 8x10⁷ ml⁻¹ by diluting the stationary phase culture with fresh nutrient broth.

### Preparation of titration curve for amending nutrient broth at different pH settings

In order to produce nutrient broth at different pH settings for the assay, a titration curve for the pH of the acid solution mixed with nutrient broth (v/v) was produced by mixing different ratios of nutrient broth and 0.5M HCl.

The antibacterial activity of acidified nitrite was carried out in wells of microtitre plates having a volume of 240 µl. Rather than measure the pH at such a small volume, the total volume of the 0.5 M HCl and nutrient broth mixture for the titration curve was multiplied 100 fold. The pH was measured using a pH meter (CD720 WPA UK) after mixing HCl and nutrient broth at different ratios to a total volume of 24 ml in a glass universal.

The pH value for each ratio of acid solution and nutrient broth mixture was obtained from 5 repeat experiments. The titration curve was plotted according to the average pH measured and volume of 0.5M HCl needed to reach the pH. This titration curve was used for the calculation of volume of 0.5 M HCl needed for 24 ml nutrient broth at certain pH setting. The actual volume of 0.5 M HCl needed to adjust 240 µl nutrient broth in the microtitre plate assay was obtained by dividing the calculated value for 24 ml by 100.

### Determination of the antibacterial activity of acidified nitrite

Experiments were carried out in sterile 96 micro titre (well) plates (Camlab, Cambridge, UK). The reaction plate was prepared by combining three separately prepared nutrient broth solutions in micro wells by pipette transfer with multichannel pipettes. During incubation the wells were only covered with a lid (no film).
The following solutions were used in each well:

| | |
|---|---|
| 1. Potassium nitrite adjusted nutrient broth*: | 60µl |
| 2. Nutrient broth with bacterial inoculum**: | 60µl |
| 3. pH adjusted nutrient broth***: | 120µl |

| | |
|---|---|
| * Potassium nitrite was prepared in distilled water and autoclaved separately. It was mixed with concentrated nutrient broth (the concentration used was to compensate the dilution of nitrite solution when mixing), just before assay, to produce normal nutrient broth concentration and different concentrations of nitrite. ** Bacteria inoculum was diluted to desired density with reference to the relevant turbidity curve. *** The pH adjusted nutrient broth was calculated to achieve final pH, taking into account all three solutions, with reference to the titration curve. The nutrient broth was concentrated to compensate the dilution when mixing with acid to give final gradients of pH as follows: 1.7, 2, 2.3, 3, 3.3, 3.6, 4, 4.5, 5, 5.5, 6 and 7.0. | |

The exposure plate was arranged as follows:

| | | 1 | 2 | 3 | 4 | 5 | 6 | 7 | 8 | 9 | 10 | 11 | 12 |
|---|---|---|---|---|---|---|---|---|---|---|---|---|---|
| | NO₂- (µM) pH → ↓ | 1.7 | 2.0 | 2.3 | 3.0 | 3.3 | 3.6 | 4.0 | 4.5 | 5.0 | 5.5 | 6.0 | 7.0 |
| A | 0 | | | | | | | | | | | | |
| B | 10 | | | | | | | | | | | | |
| C | 30 | | | | | | | | | | | | |
| D | 100 | | | | | | | | | | | | |
| E | 300 | | | | | | | | | | | | |
| F | 1000 | | | | | | | | | | | | |
| G | 3000 | | | | | | | | | | | | |
| H | 10000 | | | | | | | | | | | | |

At intervals of 0.5, 2 and 24 hours after initial exposure to acidified nitrite in a shaker at 90 rpm at 37°C, 20 µl of the suspension from each micro well was transferred to 180µl of recovery medium (Nutrient Broth) in micro plates, and a further 10-fold dilution of the recovery medium was made using the nutrient broth media. Micro well recovery plates were incubated as described above. The inhibitory effect of acidified nitrite on bacterial growth was determined by measurement of optical density (570nm) of wells using a micro well plate reader (MRX Microplate Reader, Dynatech Products Limited, UK). The minimum inhibition concentration (MIC) and the minimum bactericidal concentration (MBC) or each strain were determined by 5 repeat experiments.

The minimum inhibitory concentration (MIC) of acidified nitrite is defined as the lowest nitrite concentration whereby no growth of microorganisms had taken places at a given pH after 24 hours. The minimum bactericidal concentration (MBC) is defined as the lowest nitrite concentration whereby no growth of microorganisms takes place after transfer into recovery medium.

### Results

### MIC

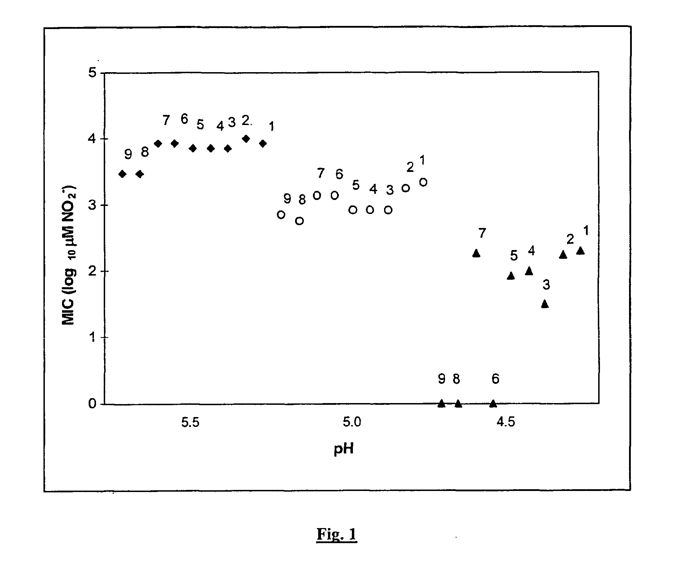

Figure 1 shows Average Minimum Inhibitory Concentrations of all clinically isolated MRSA strains, MRSA control strain, and MSSA control strain at pH 5.5 (◆), pH 5.0 (○), and pH 4.5(▲). Numbers beside each symbol represents the strain tested: 1 represents *Staph. aureus* (Methicillin-susceptible), numbers 2 to 8 represent MRSA strains A695, A762, A852, A977, A992, A1124, and A1350, respectively, and number 9 represent MRSA control strains. Mean of 5 determinations.

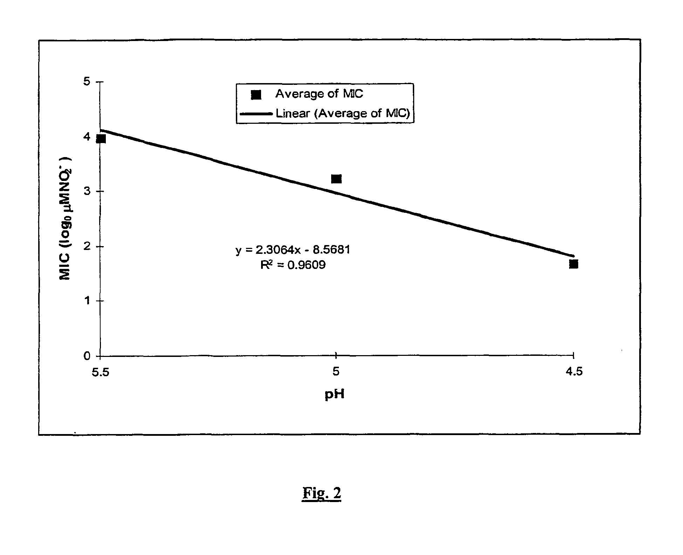

Figure 2 shows the synergistic relationship between Minimum Inhibitory Concentration of Nitrite (log₁₀ µM) and exposure acid (pH). Means of 8 strains tested.

ANOVA showed that there was no significant difference between the MIC's of all the strains tested (Figure 1, p≥ 0.05), but that the MIC's varied substantially with pH (Figure 1, p≤ 0.001). Fisher's LSD test confirmed the MIC's of all strains tested significantly decreased with the decreasing pH from 5.5 to 4.5 (Figure 2, p≤ 0.05).

There was a synergistic relationship between the logarithmic transformed MIC of nitrite and acid (pH) (Figure 2, r=0.97, p≤ 0.05).

### MBC

ANOVA showed a significant difference of MBC, according to the strain tested (p≤ 0.001). Fisher's LSD test confirmed that the MBC of acidified nitrite for *Staphylococcus aureus* (Methicillin-susceptible) was significantly ***higher*** than for two MRSA strains (A713 and A852 - p≤ 0.05), while there was no significant difference between all other strains.

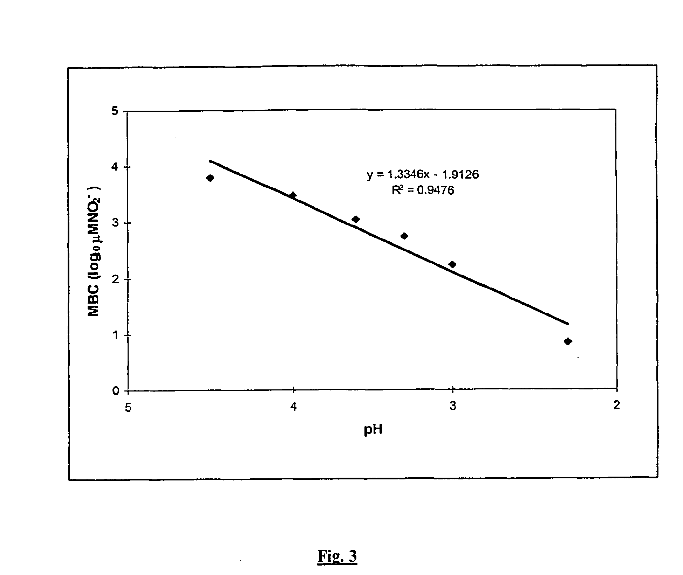

There was a synergistic relationship between logarithmic transformed Minimum Bactericidal Concentration (MBC) of nitrite and pH for all strains, but at low pH (between pH 1.7 to 2.0 for *Staphylococcus aureus,* pH 1.7 to 2.3 for all the other strains), this relationship could not be observed because, at this low pH range, bacteria were killed, even without added nitrite. A linear synergistic relationship between logarithmic transformed MBC and pH for all strains was observed (pH 2.3 - 4.5, nitrite 86 - 4556 µM - Figure 3). Figure 3 shows the synergistic relationship between Minimum Bactericidal Concentration of Nitrite (log₁₀ µM ) and exposure acid (pH). Means of 5 determinations of all 9 strains. Only MBC at pH 2.3 3.0, 3.3, 3.6, 4.0, 4.5 settings were used to plot figure.

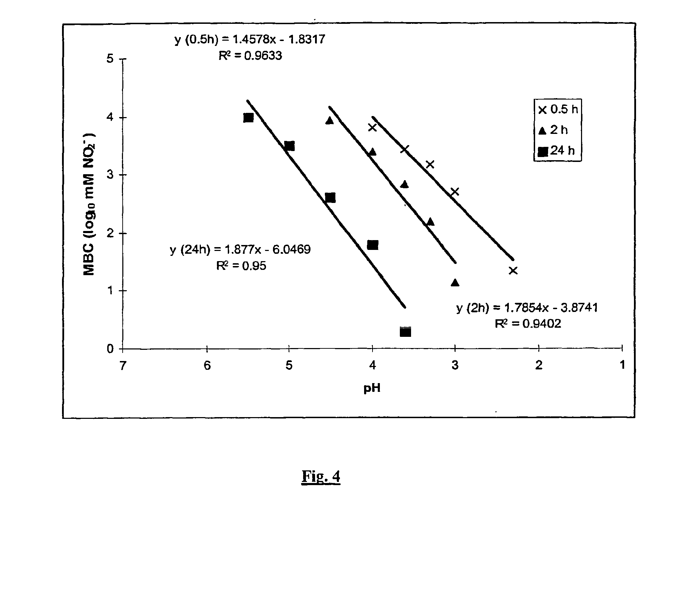

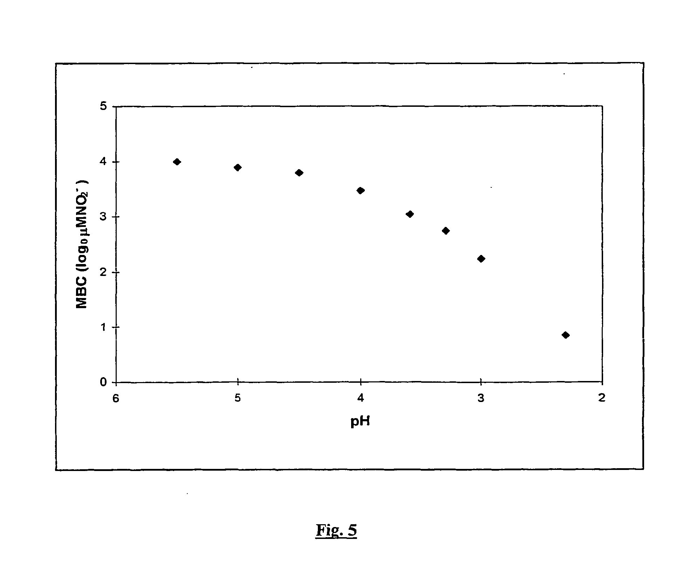

ANOVA also showed a significant difference of MBC's between different exposure time for each strains (p≤ 0.001). Figure 4 compares the MBC's of acidified nitrite at different exposure time for all strains tested. Means of 5 determinations for 9 strains. This Figure shows the relationships between MBC, pH, and different exposure times for all strains. Fisher's LSD test confirmed the MBC's of acidified nitrite followed the order of 0.5h > 2h > 24h for all strains (Figure 4 - p≤ 0.05). Figure 5 shows the synergistic relationship between Minimum Bactericidal Concentration of Nitrite (log₁₀ µM) and exposure acid (pH). Means of 5 determinations of all 9 strains. Only MBC between pH 2.3 and 5.5 settings were used to plot the figure.

There was no significant difference in the Minimum Inhibitory Concentration of acidified nitrite for MSSA, MRSA clinical isolates, and the MRSA control strain. These results indicate that acidified nitrite is capable of inhibiting MRSA strains as efficiently as MSSA strains. All MRSA strains required a Minimum Bactericidal Concentration (MBC) either lower than, or similar to, the MBC for the MSSA strain, thereby showing that acidified nitrite kills MRSA strains at least as efficiently it kills MSSA strains, if not more so.

The MBC's of acidified nitrite for different exposure times were significantly different, with the longer the exposure time, the lower the MBC of acidified nitrite needed in general. This was surprising, as most of the NO production is complete after 1hour, so that increased killing at 2 hours and 24 hours was not expected. This time dependency may indicate a sink effect, whereby NO donors are formed and subsequently slowly release NO.

### EXAMPLE 3

### Formulations

### 1. Cream formulation for sodium nitrite 0.5 to 9% and citric acid 0.75-13.5%

| **UNIT DOSE FORMULA:** (% w/w) | **INGREDIENT:** | (%w/w) | |
|---|---|---|---|
| 0.5 to 9% | SODIUM NITRITE | 0.75-13.5% | CITRIC ACID MONOHYDRATE |
| 5.00 | ARLATONE 983S | 5.00 | ARLATONE 983S |
| 0.30 | DIMETHICONE | 0.30 | DIMETHICONE |
| 1.50 | CETYL ALCOHOL | 1.50 | CETYL ALCOHOL |
| 5.00 | HEAVY LIQUID PARAFFIN | 5.00 | HEAVY LIQUID PARAFFIN |
| 9.00 | WHITE SOFT PARAFFIN | 9.00 | WHITE SOFT PARAFFIN |
| Adjust with SN¹ content approx 60% | WATER | Adjust for CA² Content approx 50- 60% | WATER |
| 15.00 | PROPYLENE GLYCOL | 15.00 | PROPYLENE GLYCOL |

| | | | |
|---|---|---|---|
| ¹ - Sodium nitrite ² - Citric acid monohydrate | | | |

### SODIUM NITRITE FORMULATION

### PREPARATION OF WAXY PHASE

Melt the following at 70 - 80°C:
Cetyl alcohol
Arlatone 983S

Wait until approximately 95% of the mixture has melted before adding the following ingredients with gentle stirring:
Dimethicone
Heavy liquid paraffin
White soft paraffin
Propylene Glycol
while maintaining the temperature at 60 - 70°C.

When all the ingredients have melted mix until homogeneous.

### PREPARATION OF AQUEOUS PHASE

Heat the following to 70 - 80°C:
Water
Sodium nitrite
Add sodium nitrite to the water and maintain the aqueous phase at this temperature until it is mixed with the waxy phase.
When both phases are ready mix them together and continuously homogenising using the Silverson mixer until the cream base has cooled to ca 60°C.
Add to the cream to the aqueous phase whilst homogenising using the Silverson mixer or similar. Continue homogenising for 10 minutes.
Transfer the cream to a small Hobart and mix continually at slow speed until the cream has set.

Transfer the finished product into a suitable container(s) ready for filling into a dual dispenser.

### CITRIC ACID FORMULATION

Prepared as for the sodium nitrite component, above.

### 2. Ointment

| Sodium Nitrite or Citric acid monohydrate | 0.5-9% w/w or 0.75- 13.5%, respectively |
|---|---|
| Cetomacrogol 1000 BP | 1.80% w/w |
| Cetostearyl Alcohol BP | 7.20% w/w |
| Liquid Paraffin BP | 6.00% w/w |
| White Soft Paraffin BP | 15.00% w/w |
| Water | Approx 70% |

### Method of manufacture

### PREPARATION OF WAXY PHASE

Melt the following at 60-70°C:
Cetyl alcohol
Cetomacrogol 1000
Wait until approximately 95% of the mixture has melted before adding the following ingredients with gentle stirring:
Liquid paraffin and White soft paraffin
Maintain the temperature at 60 - 70°C.
When all the ingredients have melted, mix until homogeneous.

### PREPARATION OF AQUEOUS PHASE

Heat the following to 60 - 70°C:
Water and add slowly the sodium nitrite or citric acid monohydrate, as appropriate, and maintain the aqueous phase at this temperature until it is mixed with the waxy phase.
When both phases are ready, mix them together and continuously homogenise, using the Silverson mixer, until the cream base has cooled to *ca.* 60°C.
Add the cream to the aqueous phase whilst homogenising using the Silverson mixer or similar. Continue homogenising for 10 minutes.
Transfer the cream to a small Hobart and mix continually at slow speed until the cream has set.

### 3. Lotion

| Sodium Nitrite or Citric acid monohydrate | 0.5-9% w/w or 0.75- 13.5%, respectively |
|---|---|
| Glycerol | 50 |
| Cetostearyl Alcohol BP | 30 |
| Water | Approx to 100% depending upon CA or SN concentration |

Heat water and add sodium nitrite or citric acid monohydrate.

### 4. Solution

| Sodium Nitrite or Citric acid monohydrate | 0.5-9% w/w or 0.75-13.5%, respectively |
|---|---|
| Water Ph Eur | 91-99.5% or 86.5-99.25 |
| parabens | 0.015-0.2% |

### Method of manufacture

Heat water to about 50°C, add sodium nitrite and paraben and dissolve.

### 5. Wax

The two components of a wax formulation were made up as follows (% by weight):

### A) 10% ASCORBYL PALMITATE

Component

| | |
|---|---|
| Ascorbyl Palmitate | 10% |
| White Soft Paraffin | 25 |
| Light Liquid Paraffin | 20 |
| Hard Paraffin | 20 |
| Arlacel 165 | 15 |
| Cetosteryl Alcohol | 10 |

### Method

1. Weigh all the components into a vessel.
2. Heat the vessel and stir the mixture until all the components have melted and the mixture is homogeneous.
3. Pour the molten wax into jars and allow to cool to room temperature.

### B) 10% SODIUM NITRITE WAX

Components

| | |
|---|---|
| Phase A | |
| Light Liquid Paraffin | 7.5% |
| White Soft Paraffin | 20 |
| Arlacel 582 | 10 |
| Cetosteryl alcohol | 10 |
| Phenoxyethanol | 1 |
| | |
| Phase B | |
| Sodium Nitrite | 10 |
| Purified Water | 20 |

### Method

1. Weigh the Phase A components into a vessel, heat to 70°C and stir until homogeneous.
2. Weigh the Phase B components into another vessel, heat to 70°C and stir, ensuring that all the sodium nitrite has dissolved.
3. When both phases have reached 70°C, add phase A to phase B and homogenise for 5 minutes.
4. Pour the molten wax into jars and allow to cool to room temperature.

## Claims

1. Use of a nitrogen oxide generating composition in the manufacture of a medicament for the treatment and/or prophylaxis of a Methicillin Resistant *S. aureus* (MRSA) infection of a subject, wherein the medicament is for topical application and is provided as at least two components for admixture, either immediately prior to application or *in situ,*
essential reactants for said nitrogen oxide generation being separately disposed in said components, such that there is substantially no nitrogen oxide generation until admixture.

2. Use according to claim 1, wherein a first essential reactant is a pharmaceutically acceptable nitrite and a second essential reactant is a pharmaceutically acceptable acidifying agent.

3. Use according to claim 1 or 2, wherein an essential reactant is an organic acid.

4. Use according to any preceding claim, wherein the medicament is adapted for administration to a site selected from the group consisting of nasal passages, mucus membranes, orthopaedic pin wounds, pressure sores, catheter associated punctures, burns and surface traumas.

5. Use according to any preceding claim, wherein the pH of the medicament is below 5 immediately after admixture.

6. Use according to claim 5, wherein the pH immediately after admixture is below 4.

7. Use according to any preceding claim, wherein the medicament comprises alkali metal or alkali earth metal nitrite in an amount of 0.005 to 15% by volume of the medicament.

8. Use according to any preceding claim, wherein the medicament comprises acidifying agent in an amount of 0.5 to 22% by volume.

9. Use according to any preceding claim, wherein the medicament, when admixed, is aqueous.

10. Use according to any preceding claim, wherein the medicament is in a form individually selected from the group consisting of solutions, washes, emulsions, suspensions, colloids, foams, sprays, gels, creams, ointments, tinctures, unguents, soft waxes, lotions, dressings and patches.

11. Use according to any preceding claim, wherein a first component of the medicament is a semi-solid suitable for topical administration, and a second component is a patch comprising a matrix containing an essential reactant suitable for applying over the gel thereby to permit contact of the reactants.

12. Use according to any preceding claim, wherein a second component is selected from the group consisting of formic acid, acetic acid, malic acid, lactic acid, citric acid, benzoic acid, tartaric acid, salicylic acid, ascorbic acid and ascorbyl palmitate.

13. Use according to any preceding claim, wherein citric acid and either sodium nitrite or potassium nitrite are used in a molar ratio of 1.2 : 1 to 2.5 : 1, by weight.

14. Use according to claim 13, wherein the acid and nitrite are used in a ratio of from 1.5:1 to 2:1.

15. Use of a nitrogen oxide generating composition in the manufacture of a medicament for the treatment and/or prophylaxis of a Methicillin Resistant *S. aureus* (MRSA) infection of a subject, wherein the medicament is for topical application and is provided as at least two components for admixture, either immediately prior to application or *in situ,*
essential reactants for said nitrogen oxide generation being separately disposed in said components, such that there is substantially no nitrogen oxide generation until admixture,
the medicament being for co-administration with at least one antibiotic suitable for treatment of non-resistant strains of said infection.

16. Use according to claim 15, wherein the antibiotic is one to which the infection was resistant until treatment with NO/NO₂.

17. Use according to claim 15 or 16, wherein the antibiotic is penicillin or a cephalosporin.

18. Use according to any of claims 1 to 17, wherein there are two components in the form of dry powders, the nitrogen oxide generating composition being formed when the powders are combined in the presence of water.

## Patentansprüche

1. Verwendung einer Stickstoffoxid bildenden Zusammensetzung bei der Herstellung eines Medikaments für die Behandlung und/oder Präventivbehandlung einer gegen Methicillin resistenten S. Aureus- (MRSA-) Infektion eines Patienten, wobei das Medikament der topischen Anwendung dient und als mindestens zwei Komponenten zum Vermischen entweder direkt vor der Anwendung oder in situ bereitgestellt wird,
wobei die wesentlichen Reaktanden für die Stickstoffoxidbildung getrennt innerhalb der Komponenten derart vorliegen, dass im Wesentlichen keine Stickstoffoxidbildung bis zum Vermischen stattfindet.

2. Verwendung nach Anspruch 1, wobei ein erster wesentlicher Reaktand ein pharmazeutisch akzeptables Nitrit und ein zweiter wesentlicher Reaktand ein pharmazeutisch akzeptables Ansäuerungsmittel ist.

3. Verwendung nach Anspruch 1 oder 2, wobei ein wesentlicher Reaktand eine organische Säure ist.

4. Verwendung nach einem vorhergehenden Anspruch, wobei das Medikament für die Verabreichung an einer Stelle geeignet ist ausgewählt aus der Gruppe bestehend aus Nasengängen, Schleimhäuten, durch orthopädische Nägel verursachten Wunden, Dekubitus, mit Katheter assoziierten Einstichen, Branntwunden und Oberflächentraumas.

5. Verwendung nach einem vorhergehenden Anspruch, wobei der pH-Wert des Medikaments direkt nach dem Vermischen unter 5 lieg.

6. Verwendung nach Anspruch 5, wobei der pH-Wert direkt nach dem Vermischen unter 4 liegt.

7. Verwendung nach einem vorhergehenden Anspruch, wobei das Medikament Alkalimetall- oder Erdalkalimetallnitrit in einer Menge von 0,005 bis 15 Volumen-%, auf das Medikament bezogen, umfasst.

8. Verwendung nach einem vorhergehenden Anspruch, wobei das Medikament Ansäuerungsmittel in einer Menge von 0,5 bis 22 Volumen-% umfasst.

9. Verwendung nach einem vorhergehenden Anspruch, wobei das Medikament nach dem Vermischen wässrig ist.

10. Verwendung nach einem vorhergehenden Anspruch, wobei das Medikament in einer Form vorliegt, die einzeln aus einer Gruppe ausgewählt ist bestehend aus Lösungen, Spülflüssigkeiten, Emulsionen, Suspensionen, Kolloiden, Schäumen, Sprays, Gelen, Cremes, Salben, Tinkturen, Unguenta, weichen Wachsen, Lotionen, Verbänden und Pflastern.

11. Verwendung nach einem vorhergehenden Anspruch, wobei eine erste Komponente des Medikaments eine halbfeste Substanz ist, die für die topische Anwendung geeignet ist, und eine zweite Komponente ein Pflaster ist, die eine Matrix die einen wesentlichen Reaktand enthält, die für das Aufbringen über dem Gel geeignet ist, um **dadurch** den Kontakt der Reaktanden zu gestatten.

12. Verwendung nach einem vorhergehenden Anspruch, wobei eine zweite Komponente aus der Gruppe ausgewählt ist bestehend aus Ameisensäure, Essigsäure, Apfelsäure, Milchsäure, Zitronensäure, Benzoesäure, Weinsäure, Salicylsäure, Ascorbinsäure und Ascorbylpalmitat.

13. Verwendung nach einem vorhergehenden Anspruch, wobei Zitronensäure und entweder Natriumnitrit oder Kaliumnitrit in einem molaren Verhältnis von 1,2 : 1 bis 2,5 : 1, auf das Gewicht bezogen, verwendet werden.

14. Verwendung nach Anspruch 13, wobei die Säure und das Nitrit in einem Verhältnis von 1,5 : 1 bis 2 : 1 verwendet werden.

15. Verwendung einer Stickstoffoxid bildenden Zusammensetzung bei der Herstellung eines Medikaments für die Behandlung und/oder Präventivbehandlung einer gegen Methicillin resistenten S. Aureus- (MRSA-) Infektion eines Patienten, wobei das Medikament der topischen Anwendung dient und als mindestens zwei Komponenten zum Vermischen entweder direkt vor der Anwendung oder in situ bereitgestellt wird,
wobei die wesentlichen Reaktanden für die Stickstoffoxidbildung getrennt innerhalb der Komponenten derart vorliegen, dass im Wesentlichen keine Stickstoffoxidbildung bis zum Vermischen stattfindet,
wobei das Medikament der gleichzeitigen Verabreichung mit mindestens einem Antibiotikum dient, das für die Behandlung nichtresistenter Stämme der Infektion geeignet ist.

16. Verwendung nach Anspruch 15, wobei das Antibiotikum eines ist, gegen das die Infektion bis zur Behandlung mit NO/NO₂ resistent gewesen ist.

17. Verwendung nach Anspruch 15 oder 16, wobei das Antibiotikum Penicillin oder ein Cephalosphorin ist.

18. Verwendung nach einem der Ansprüche 1 bis 17, wobei zwei Komponenten in Form von trockenen Pulvern vorliegen, wobei die Stickstoffoxid bildende Zusammensetzung dann gebildet wird, wenn die Pulver in Gegenwart von Wasser kombiniert werden.

## Revendications

1. Utilisation d'une composition générant un oxyde d'azote dans la fabrication d'un médicament pour le traitement et/ou la prophylaxie d'une infection de *S. aureus* résistant à la méthicilline (MRSA) d'un sujet, dans laquelle le médicament est pour une application topique et il est fourni sous forme d'au moins deux composants pour un mélange, soit immédiatement avant l'application, soit in situ,
les réactifs essentiels pour ladite génération d'oxyde d'azote étant disposés séparément dans lesdits composants, de sorte qu'il n'y a substantiellement pas de génération d'oxyde d'azote avant le mélange.

2. Utilisation suivant la revendication 1, dans laquelle un premier réactif essentiel est un nitrite pharmaceutiquement acceptable et un deuxième réactif essentiel est un agent acidifiant pharmaceutiquement acceptable.

3. Utilisation suivant la revendication 1 ou 2, dans laquelle un réactif essentiel est un acide organique.

4. Utilisation suivant l'une quelconque des revendications précédentes, dans laquelle le médicament est adapté pour une administration vers un site choisi dans le groupe constitué de voies nasales, de membranes mucosales, de blessures de broches orthopédiques, d'escarres de décubitus, de perforations associées à un cathéter, de brûlures et de traumas de surface.

5. Utilisation suivant l'une quelconque des revendications précédentes, dans laquelle le pH du médicament est en dessous de 5 immédiatement après le mélange.

6. Utilisation suivant la revendication 5, dans laquelle le pH immédiatement après le mélange est en dessous de 4.

7. Utilisation suivant l'une quelconque des revendications précédentes, dans laquelle le médicament comprend un nitrite de métal alcalin ou de métal alcalino-terreux dans une quantité de 0,005 à 15% en volume du médicament.

8. Utilisation suivant l'une quelconque des revendications précédentes, dans laquelle le médicament comprend un agent acidifiant dans une quantité de 0,5 à 22% en volume.

9. Utilisation suivant l'une quelconque des revendications précédentes, dans laquelle le médicament, lorsqu'il est mélangé, est aqueux.

10. Utilisation suivant l'une quelconque des revendications précédentes, dans laquelle le médicament est sous une forme choisie individuellement dans le groupe constitué de solutions, de lavages, d'émulsions, de suspensions, de colloïdes, de mousses, de sprays, de gels, de crèmes, d'onguents, de teintures, de pommades, de cires molles, de lotions, de pansements et de patchs.

11. Utilisation suivant l'une quelconque des revendications précédentes, dans laquelle un premier composant du médicament est un semi-solide approprié pour une administration topique et un deuxième composant est un patch comprenant une matrice contenant un réactif essentiel approprié pour une application sur le gel, de manière à permettre un contact des réactifs.

12. Utilisation suivant l'une quelconque des revendications précédentes, dans laquelle un deuxième composant est choisi dans le groupe constitué d'acide formique, d'acide acétique, d'acide malique, d'acide lactique, d'acide citrique, d'acide benzoïque, d'acide tartrique, d'acide salicylique, d'acide ascorbique et de palmitate d'ascorbyle.

13. Utilisation suivant l'une quelconque des revendications précédentes, dans laquelle de l'acide citrique et soit du nitrite de sodium, soit du nitrite de potassium sont utilisés en un rapport molaire de 1,2:1 à 2,5:1, en poids.

14. Utilisation suivant la revendication 13, dans laquelle l'acide et le nitrite sont utilisés en un rapport de 1,5:1 à 2:1.

15. Utilisation d'une composition générant un oxyde d'azote dans la fabrication d'un médicament pour le traitement et/ou la prophylaxie d'une infection de S. *aureus* résistant à la méthicilline (MRSA) d'un sujet, dans laquelle le médicament est pour une application topique et il est fourni sous forme d'au moins deux composants pour un mélange, soit immédiatement avant l'application, soit in situ,
les réactifs essentiels pour ladite génération d'oxyde d'azote étant disposés séparément dans lesdits composants, de sorte qu'il n'y a substantiellement pas de génération d'oxyde d'azote avant le mélange,
le médicament étant pour une co-administration avec au moins un antibiotique approprié pour le traitement de souches non résistantes de ladite infection.

16. Utilisation suivant la revendication 15, dans laquelle l'antibiotique est un composé auquel l'infection était résistante jusqu'à un traitement avec NO/NO₂.

17. Utilisation suivant la revendication 15 ou 16, dans laquelle l'antibiotique est une pénicilline ou une céphalosporine.

18. Utilisation suivant l'une quelconque des revendications 1 à 17, dans laquelle il y a deux composants sous la forme de poudres sèches, la composition générant un oxyde d'azote étant formée lorsque les poudres sont combinées en présence d'eau.
